# EUROPEAN PATENT APPLICATION

(11) **EP 1 810 655 A2**
(43) Date of publication of application: **25.07.2007**
(21) Application number: 07001001.2
(22) Date of filing: 18.01.2007
(51) Int. Cl.: A61K 6/00, A61Q 11/00

(54) **Dental composition for root canal treatment**

(30) Priority: 24.01.2006 IT PI20060007
(71) Applicant: ITALMED S.r.l., 50132 Firenze (IT)
(72) Inventor: Tosetti, Carlo, 50014 Fiesole (FI) (IT)
(74) Representative: Celestino, Marco

(57) **Abstract**

Dental composition for root canal treatment comprising an aqueous disinfecting solution, deionized water and a tixotropic substance, such as hydrated silica, or metal silicates. The aqueous disinfecting solution is selected from the group comprised of: a solution of sodium hypochlorite, of calcium hypochlorite, of potassium hypochlorite, of magnesium hypochlorite, lithium hypochlorite, of potassium isocyanurate dichloride, of sodium isocyanurate dichloride, of dihydrated sodium isocyanurate dichloride, of trichloridecyanuric acid, of 1,3-dichloride-5,5-dimethylhydantoin, of N-chlorosulphamide, T chloramine, of T dichloramine, of B chloramine and of B dichloramine, etc. Owing to the alkaline properties of the chosen tixotropic substance it is possible, in particular, to use an amount of pH regulator less than compositions of prior art for bringing the pH of the solution to a suitable range of values, i.e. obtaining a very high steadiness of the disinfecting solution.

## Description

### Field of the invention

The present invention relates to the field of endodontics, and, in particular, it relates to a dental composition for root canal treatment.

### Description of the prior art

As well known, endodontics is the field of dentistry that relates to treatment of the inner portion of teeth, i.e. dental pulp and root canals. In particular, the dental pulp extends from the pulp chamber through the root canal up to apex of the root of a tooth. This consists of a soft tissue of the tooth containing blood vessels, connective tissue, collagen, and nervous tissue. When the tooth is damaged by a deep caries, traumatic or other causes, bacteria and their toxins can penetrate the dental pulp causing an inflammation, or pulpitis, which can bring to the loss of vitality of the tooth. Furthermore, a tooth with an infected or sick pulp is particularly affected by changes of temperature and is subject to decoloration. Therefore, in such cases a treatment is necessary, i.e. for distancing the dental pulp in the pulp chamber and the tissue contained in the root canals, for avoiding that the infection extends to periapical bone tissues and then proceeds at a systemic level, as well as, in an acute phase, for soothing pain of the patient.

The dental pulp can be removed by extracting the tooth, when the structure of the tooth same is irremediably compromised, or resorting to endodontics, by a root canal treatment described hereafter.

The endodontic treatment, or devitalization, consists of removing the dental pulp, comprising bacteria and their toxins, from the pulp chamber and from the root canal. It begins with a step of making an opening, by means of appropriate cutters mounted on a drill, through the tooth crown in order to reach the pulp chamber. Then, the pulp is withdrawn, the root canal cleaned, enlarged and prepared for being filled and sealed permanentely with a biocompatible material, usually gutta-percha and specific root canal fillings.

However, the systems commonly used for carrying out a root canal treatment provide the use of tools, such as root canal files, which, even if very thin cannot reach completely the channel internal walls where bacteria can be present. Therefore, sometimes it is necessary to repeat the root canal treatment also a long time after the first treatment with considerable discomfort for the patient.

Many bacteria disinfectants are used during the endodontic treatment. Known disinfectants are liquid, presumably to make easier the penetration of the product up to reaching generally the channel sides and the dentinal tubules. However, all the disinfecting/irrigating fluids, even the most active, become ineffective in the eradication of bacterial infection in 10% of all the root canal treatments.

Furthermore, such disinfectants have relevant problems, particularly if the root canal treatment has to be carried out on a tooth that belongs to the upper dental arch. In fact, force of gravity causes the liquid composition to fall from the root canals with loss of efficiency of the treatment and with the risk of polluting the mouth causing irritation, burnings, tendency to choking and troubles of various types. The dentist is therefore forced to lie the patient on the back with hyper-extension of the head for limiting the fall of the disinfectant from the tooth chamber. Even the possible use of rubber barriers is not completely effective in the control the potential fall of disinfectant and then the irritation of the oral neighbouring tissues.

Furthermore, such liquid disinfectants do not have any lubricant properties, which would be instead required to assist the penetration of the root canal files, for reducing the production of dentin "plugs" that can form during the root canal treatment, and for reducing the risk of damageing the files. Such root canal files, which can be used manually or mounted on a micromotore, have, in fact, a high risk of breaking in the canals.

Disinfectants also exist having high viscosity, with a consequent difficulty to reach effectively even the portions of the canals that are of very small size.

The above described problems are in part soved by the dental composition for root canal treatment described in US 2003/0156980A1. This composition comprises, in particular, an aqueous disinfecting solution sodium hypochlorite and "fumed silica" (SiO2), i.e. a particular structure of silice anhydrous, as thickener. This composition, which firstly is liquid, increases gradually its own viscosity up to providing a gel phase. This way, the disinfecting composition can adhere to the walls of the treated tooth to solve some of the above cited problems, among which the need for arranging the patient in position lying on the back by head hyper-extension during the root canal treatment.

However, fumed silica in aqueous dispersion has a very acid pH, approximately between 3 and 5. Therefore, in order to provide an highly basic environment (pH set between 12 and 13), which is necessary for making steadier the solution of sodium hypochlorite, a high amount of a pH regulator has to be added. This causes, as demonstrated in many studies, a considerable loss of rheologic steadiness of the composition and of chloride title steadiness.

### Summary of the invention

It is therefore a feature of the invention to provide a composition for endodontic therapies for carrying out an effective root canal treatment and that causes in the meantime a minimum discomfort to the patient.

It is another feature of the invention to provide a composition for endodontic therapies that assists the root canal treatment with respect to normally used techniques and for assisting the operation also on teeth of the upper dental arch in a safer way.

It is another feature of the invention to provide a composition for endodontic therapies capable of assuring a high rheologic steadiness and a chloride title steadiness much higher than in the compositions of prior art.

It is a further feature of the invention to provide a composition for endodontic therapies that requires low amounts of pH regulator to ensure optimal operation conditions.

These and other features are accomplished with one exemplary dental composition for root canal treatment, according to the invention, comprising:
- an aqueous disinfecting solution,
- deionized water,
- a thickening agent,
wherein the thickening agent is a tixotropic substance selected from the group comprised of:
- precipitated silica,
- a metal silicate,
- A combination thereof.

Preferably, the metal silicate is selected from the group comprised of:
- calcium silicate,
- aluminum silicate,
- a combination thereof.

In particular, the precipitated silica may have a composition of the type SiO2•nH2O, i.e. can be a hydrated silica with hydratation rate n, in particular with n set between 1 and 9.

As above stated, the thickening agent is a tixotropic substance i.e. capable of changing the physical status of a liquid increasing the static viscosity and maintaining low the dynamic viscosity. The ratio between the two values goes to the tixotropic index. This feature assists the application of the gel, since the low dynamic viscosity keeps the composition fluid while it is in movement during the application step, to pass immediately in the state of high viscosity as it stops on a surface. Therefore, the viscosity of the composition, according to the invention, allows keeping the disinfecting solution at a direct contact of the walls of the root canals in order to provide a deep and effective disinfecting action.

Test applications have shown that the thickening agent of the invention allow to keep practically unchanged the rheologic features of the gel also for long periods, about 2 years. As well known, in fact, in the presence of strongly oxidant substances, like sodium hypochlorite, the gelifying substances alter progressively with time and then change the rheologic features of the gel same, which looses progressively its viscosity. The thickening substances chosen according to the invention provide also a further advantage, i.e. that rheological stabilizers are not required, such as, for example polyethylen glycol, glycerin, propylen glycol, polypropylen glycol, sorbitol, which would have quickly jeopardized the steadiness of the sodium hypochlorite.

The composition, according to the invention, is highly steady, concerning in particular the content of sodium hypochlorite. The chloride title remains substantially fixed, decreasing after two years not much below the starting percentage. To this purpose, it is instead known the extreme unsteadiness of the sodium hypochlorite both in liquid and in gel phase, as used in the compositions of prior art.

In particular, the thickening agent is present in the composition of the invention in a concentration set between 0.1% and 40% of the overall weight of the composition, advantageously between 0.5% and 30%, preferably between 5% and 28% of the overall weight of the composition.

Advantageously, the thickening agent is selected from the group comprised of: precipitated silica, and silicates of aluminium or calcium, or a combination thereof.

Preferably, the thickening agent used is aluminum silicate.

Advantageously, the precipitated silica and the silicates used are known with the trademark "Sipernat", in particular, Sipernat 500 LS, Sipernat 360, Sipernat 820A, Sipernat 880.

Advantageously, the aqueous disinfecting solution is selected from the group comprised of: a solution of sodium hypochlorite, of calcium hypochlorite, of potassium hypochlorite, of magnesium hypochlorite, lithium hypochlorite, of potassium isocyanurate dichloride, of sodium isocyanurate dichloride, of dihydrated sodium isocyanurate dichloride, of trichloroisocyanuric acid, of 1,3-dichloride-5,5-dimethylhydantoin, of N-chlorosulphamide, of T chloramine, of T dichloramine, of B chloramine and of B dichloramine.

Preferably, the aqueous disinfecting solution is a solution of sodium hypochlorite.

In particular, the solution of sodium hypochlorite is used in a concentration suitable to ensure both the disinfecting functions and the functions of "digestion/dissolution" of the collagenic component present in the root canals. More in detail, the sodium hypochlorite is present in the composition in a concentration set between 0.1% and 15% of the overall weight of the composition, advantageously set between 0.5% and 10% of the overall weight of the composition, and preferably in an amount set between 1% and 8% of the overall weight of the composition.

As well known, the steadiness of the sodium hypochlorite increases responsive to pH. Therefore, a pH regulator adapted to fix the pH of the aqueous disinfecting solution, in particular, of sodium hypochlorite, maintaining it in a determined range, is provided.

In particular, the pH regulators maintain the pH of the aqueous disinfecting solution at a value larger than 10, advantageously at a value larger than 11, preferably at a value larger than 12.

The pH regulator may be selected from the group comprised of: sodium hydroxide, potassium hydroxide, triethanolamine. In particular, the amount of pH regulator necessary for bringing the solution of sodium hypochlorite into a range of desired values is low. This because, differently from the fumed silica, which when dispersed in water is acid (pH: 3.5 ÷ 5.5), the thickening agents according to the present invention, when dispersed in water, have a alkaline pH, for example Sipernat 500 LS: pH = 6, Sipernat 360: pH=9, Sipernat 820A: pH=9,7, and Sipernat 880: pH= 10,5. This minimum supply of a pH regulator is advantageous since it is known that an excessive addition of solutes to a gel reduces the rheologic steadiness.

With reference to the hydrated precipitated silica (SiO₂.nH₂O), used in present invention, it is made by a mixing reaction at an alkaline pH. The production of the precipitated silica starts with the reaction between a solution of alkaline silicate and a mineral acid. The solution of sulphuric acid and sodium silicate are added to water at the same time under stirring. The result is a white precipitate that will then be filtered, washed and dried. The precipitated silica dispersed in water (1 to 20) has a pH from 4 to 8.

The fumed silica (SiO₂) claimed in US 2003/0156980A1 cited in the introductory part is made by hydrolysis of the vapour phase of a silicon halogenure like silicon tetrachloride. This means that it is obtained as final product of a process carried out at high temperature (1000°C for 2 hours).

The invention will now be described in a way not limitative, through the following examples.

### Example 1

Aluminum silicate (Sipernat 820A) 5-40%
Sodium hypochlorite 0,5-15%
Sodium hydroxide (sol. 1:1) up to pH 11,1
Deionized water up to 100

### Example 2

Precipitated silica (Sipernat 500LS) 5-15%
Sodium hypochlorite 0,5-15%
Sodium hydroxide (sol. 1:1) up to pH 11,4
Deionized water up to 100

### Example 3

Precipitated silica (Sipernat 360) 5-25%
Sodium hypochlorite 0,5-15%
Sodium hydroxide (sol. 1:1) up to pH 11,2
Deionized water up to 100

### Example 4

Calcium silicate (Sipernat 880) 5-40%
Sodium hypochlorite 0,5-15%
Sodium hydroxide (sol. 1:1) up to pH 11,2
Deionized water up to 100

### Example 5

Aluminum silicate (Sipernat 820A) 5-30%
Precipitated silica (Sipernat 500LS) 5-10%
Sodium hypochlorite 0,5-15%
sodium hydroxide (sol. 1:1) up to pH 11,5
deionized water up to 100

### Example 6

Precipitated silica (Sipernat 360) 5-15%
Precipitated silica (Sipernat 500LS) 5-10%
Sodium hypochlorite 0,5-15%
Sodium hydroxide (sol. 1:1) up to pH 11,5
Deionized water up to 100

### Example 7

Aluminum silicate (Sipernat 820A) 5-30%
Calcium silicate (Sipernat 880) 5-30%
Sodium hypochlorite 0,5-15%
Sodium hydroxide (sol. 1:1) up to pH 11,5
Deionized water up to to 100

## Claims

1. Dental composition for root canal treatment comprising:
- an aqueous disinfecting solution,
- a thickening agent,
- deionized water,
**characterised in that** said thickening agent is a tixotropic substance selected from the group comprised of:
- precipitated silica,
- metal silicate,
- a combination thereof.

2. Composition, according to claim 1, wherein said metal silicate is selected from the group comprised of:
- calcium silicate,
- aluminum silicate,
- a combination thereof.

3. Composition, according to claim 1, wherein said aqueous disinfecting solution is selected from the group comprised of:
- a solution of sodium hypochlorite,
- a solution of calcium hypochlorite,
- a solution of potassium hypochlorite,
- a solution of magnesium hypochlorite,
- a solution of lithium hypochlorite,
- a solution of potassium isocyanurate dichloride,
- a solution of sodium isocyanurate dichloride,
- a solution of sodium dihydrated isocyanurate dichloride,
- a solution of trichloroisocyanuric acid,
- a solution of 1,3-dichloride-5,5-dimethylhydantoin,
- a solution of N-chlorosulphamide,
- a solution of T chloramine,
- a solution of T dichloramine,
- a solution of B chloramine
- a solution of B dichloramine.

4. Composition, according to claim 1, wherein said thickening agent is present in said composition in a concentration set between 0.1% and 40% of the overall weight of the composition, advantageously between the 0.5% and 30%, preferably set between 5% and 28% of the overall weight of the composition.

5. Composition, according to claim 1, wherein said aqueous disinfecting solution is present in a concentration set between 0.1% and 15% of the overall weight of the composition, advantageously in a concentration set between 0.5% and 10% of the overall weight of the composition, and preferably in a concentration set between 1% and 8% of the overall weight of the composition.

6. Composition, according to claim 1, wherein, furthermore, a pH regulator is provided adapted to fix the pH of said aqueous disinfecting solution maintaining it at a determined range, in particular, suitable for making a strongly alkaline solution.

7. Composition, according to claim 8, wherein said pH regulator is adapted to keep said pH of said aqueous disinfecting solution at a value larger than 10, advantageously at a value larger than 11, preferably at a value larger than 12.

8. Composition, according to claim 8, wherein said pH regulator is selected from the group comprised of:
- sodium hydroxide,
- potassium hydroxide,
- triethanolamine.
